# EUROPEAN PATENT SPECIFICATION

(11) **EP 0 917 423 B1**
(45) Date of publication and mention of the grant of the patent: **18.02.2004**
(21) Application number: 97934659.0
(22) Date of filing: 05.08.1997
(51) Int. Cl.: A01K 67/033

(54) **AQUACULTURE OF MARINE WORMS**
WASSERTIERZUCHT VON MEERWÜRMERN
AQUACULTURE DE VERS MARINS

(30) Priority: 10.08.1996 GB 9616854
(43) Date of publication of application: 26.05.1999
(60) Divisional application: 03023698.8
(73) Proprietor: Seabait Limited, Ashington, Northumberland NE63 9NW (GB)
(72) Inventor: OLIVE, Peter, James, William, Tyne and Wear NE26 4NS (GB)
(74) Representative: Pattullo, Norman
(86) International application number: PCT/GB1997/002115
(87) International publication number: WO 1998/006255

(56) References cited:
- JP-A- 52 107 998
- US-A- 3 765 372
- US-A- 4 155 331
- US-A- 4 556 010

## Description

This invention relates to aquaculture of marine worms and particularly to methods of controlling sexual maturation of polychaete worms of the Nereidae family.

Marine bait worms are animals in the Class Polychaete of the Phylum Annelida or in the Phylum Sipunculida or are such other animals as may be generally referred to as worms which may be used as bait by anglers. Such worms are also used as feedstuffs for fish crustaceans and other organisms, for toxicity testing and for other scientific purposes.

Naturally occurring supplies of marine worms are not inexhaustible and collection of marine worms has been recognised as a cause of serious environmental concern.

Aquaculture of marine worms provides a sustainable source. However, the seasonal breeding cycle of marine worms hinders provision of a constant supply of. worms throughout the year. Since the natural fecundity of female marine worms is very high, large numbers of fertilised eggs are available from time to time that are surplus to requirements and are usually wasted.

The present invention provides a method of controlling the time of sexual maturation of polychaete (Polychaeta) worms as recited in Claim 1.

Preferred features which may be used with aspects of the invention are set out in the dependent Claims.

The invention may be used as part of a worm breeding program.

In the invention the worms are held under controlled conditions of relative day and night length in a twenty four hour day, the combination of a specific day and night length being referred to as the photoperiod, and maintained in controlled conditions of temperature to provide sexually mature animals for the purposes of breeding throughout a greatly extended breeding season with or without the additional use of low temperature preserved larvae as an additional source of larvae whose effective birth date is outside the natural breeding season.

This may be achieved by exposing the animals to a predetermined sequence of changes in photoperiod according to a sequence of protocols and may be under controlled temperature conditions. The progression of gametogenesis and other components of the sexual maturation process may be so changed that the time of sexual maturation process when breeding may be carried out may be different to that observed in similar animals not subject to such rearing protocols and which may allow for the carrying out of artificial fertilisations or the induction of spawning by chemical or other means at different seasons of the year other than that of the naturally occurring breeding season. This may be achieved by means of a system for maintaining such marine or estuarine worms under controlled conditions of light and dark to simulate 24 hour photoperiodic days in which the duration of the light and dark phases can be changed from short days defined as having a light period substantially less than 12 hours to long days defined as having a light period substantially greater than 12 hours or from long days to short days. Procedures that may be adopted may be described in a series of protocols of specified photoperiods and preferred temperature that will influence the time of sexual maturation and gamete development and shall subsequently influence the times at which the animals are in a suitable state for reproduction and which with or without further treatment to induce a state in which fertilisation is possible by decerebration or exposure to late acting signals may be used for the purpose of carrying out artificial fertilisations either by the extraction of the reproductive cells from the body cavity or by the induction of breeding by exposure to chemical agents known to induce spawning.

In order to facilitate description the following terms are defined:
- Photoperiodic day:: the total duration of a single sequence of light and dark;
- Natural photoperiodic regime:: the annual sequence of photoperiodic days that occurs naturally at the location at which the animals are kept or the work is being carried out;
- Effective date of birth:: the actual date of fertilisation of an animal or the date of recovery of the animal from a cryopreservation system;
- Photophase:: the duration of a period of light in the photoperiodic day;
- Scutaphase:: the duration of dark in the photoperiodic day;
- Critical photophase:: the duration of a photophase which is such that any greater photophase will be interpreted as being a long day and which is such that any shorter photophase is interpreted as a short day and which, in the specific case of the reaction of Nereis (Neanthes) virens described herein is a photophase of 12 hours in a 24 hour day;
- Short day:: a 24 hour photoperiodic day in which the photophase is substantially less than the critical photophase length ie ( 11 hours for Nereis (Neanthes) virens for growth of eggs;
- Long day:: a 24 hour photoperiodic day in which the photophase is substantially greater than the critical photophase ie 〉 13 hours for Nereis (Neanthes) virens for growth of eggs;
- Coelomic biopsy:: a means of determining the state of sexual maturity of a worm;
- Modal diameter:: the most frequent diameter of oocytes observed by coelomic biopsy;
- Mean diameter:: the arithmetic mean diameter of oocytes observed by coelomic biopsy;
- Temperature:: The temperature of the water in which the animals are maintained with an allowance for temporary fluctuations of around 2 °C above or below the specified temperature;
- Decerebration:: The removal by surgical means of the supraoesophageal ganglion.

Embodiments utilising the present invention and relating to controlling sexual maturation of marine worms are described, by way of example only, in the following examples.

### Step 1 - Maintenance of worms prior to selection for the process

Female and male worms may be maintained in sand (or some other suitable substrate) in boxes, tanks or other suitable container that is supplied with sea water at a temperature preferably in a range of 0 °C to 30 °C plus or minus 2 °C or may be obtained by collecting the animals from suitable natural populations.

The system of maintenance may be provided with a filtration and aeration means to maintain the water in good condition for husbandry; temperature may also be controlled.

### Step 2 - Selection of worms for the execution of the process

The worms to be introduced to the culture conditions may be selected by prior coelomic biopsy or other means and the stage of sexual development and/or the effective season of birth will normally be specified according for example to one of the procedures or protocols to be described. The selected animals may be animals at any stage of development from larvae to maturing female or male worms according to the protocols.

The stage of maturity of a female worm may be determined by examining the primary oocytes in the coelomic fluid or in some cases by examining the primary oocytes retained in the ovaries that may be found in virtually all or in some instances only a small number of the segments.

In the event that the worms are female worms in the family Nereidae, the oocytes may be sampled by withdrawing a small quantity of the body cavity fluid known as the "coelomic fluid" into a hypodermic syringe or, into a glass pasteure pipette that has a specially sharpened point or into a sharpened glass capillary. A volume of coelomic fluid containing some 50 to some 1000 oocytes may be withdrawn.

In the event that the worms are male worms in the family Nereidae, the spermatocytes may be sampled by withdrawing a small quantity of the body cavity fluid known as the "coelomic fluid" into a hypodermic syringe or, into a glass pasteur pipette that has a specially sharpened point or into a sharpened glass capillary. A volume of coelomic fluid containing some 1000s of spermatocytes or spermatogonial clusters may be withdrawn.

### Step 3 - Maintenance in a specific photoperiodic regime

In order to operate the procedures it will be necessary to have arranged conditions suitable for the maintenance of the animals under conditions of known photoperiod in which the photophase and the scutaphase may be different to those occurring naturally at the place of experimentation and the operation of the process.

Containers such as tanks, concrete or brick constructions or any suitable construction or container in which marine worms may be kept should be arranged inside chambers of any suitable construction fitted with a source of tungsten or fluorescent or any other suitable light source connected to a manually or automatically controlled switch that allows the duration of the photophase and scutaphase to be controlled or alternatively the animals be kept in containers fitted with lids which may be applied or removed manually or mechanically in order to control the ingress of natural or artificial light and by any of these means to control the duration of the light phase or in any similar arrangement that enables the duration of the photophase and scutaphase to be determined and controlled.

The photoperiod and temperature protocol to be followed may be determined with reference to one or more or all of:
i. the month of birth or the month of recovery from a preservation system, such period of recovery establishing the effective birth date;
ii. the photoperiodic treatment received from the time of birth or effective time of birth;
iii. the time of year and the ambient photoperiodical cycle;
iv. the desired season for sexual maturation and breeding;
v. the stage of gametogenic development of the animal.

### Step 4 - Assay of the progress of sexual maturation and development and selection of animals for breeding and the subsequent performance of artificial fertilisations and/or induction of breeding

Worms to be selected for the purposes of breeding may be subjected to a process of examination of the stage of sexual development by a procedure involving coelomic biopsy or some other methodology as has been substantially described in Step 2.

Females may be selected for the purposes of breeding by appropriate criteria relating to the size and morphology of the oocytes, or which shall otherwise be recognised as being of a stage ready for fertilisation. For a female of the taxonomic designation Nereis (Neanthes) virens the majority of primary oocytes should have a diameter greater that 160 µm and have a cytological halo of clear cytoplasm in the cortical region.

Males may be selected for the purposes of breeding by appropriate criteria relating to the stage of cytological development of the developing germ cells, or which otherwise are selected by criteria which indicate a readiness to become active in sea water with or without stimulation by maturation inducing substances, for example, 8,11,14 eicosatrienoic acid or such other substances as may be known to induce maturation of the sperm of polychaete worms as may be extracted from the supra-oesophageal ganglion or the body fluids. A male of the taxonomic designation Nereis (Neanthes) virens may be selected by the presence of spermatozoa dissociated from the tetrads which characterise the previous stage of sexual development.

The inventors have determined that specimens of polychaete worms will respond to the effective day length to which they are exposed; that there is a critical photoperiod for this response which is a species and reaction specific photophase and/or scutaphase and which in the case of Nereis (Neanthes) virens is 12 hour light 12 hour dark in a 24 hour day; that the rate of oocyte growth is influenced by the effective day length; that the response is further moderated by real time, age and stage of maturity; and that the rate of oocyte growth determines the time at which the animals will reach a stage of sexual maturity or reach a stage at which they can become fertilisable or can be induced to spawn. The invention, in one of its aspects, relates to a series of operations by means of which polychaete worms of the Nereidae family, which may be the polychaete worm Nereis (Neanthes) virens, may be induced to become sexually mature or otherwise fertilisable at times outside the natural breeding season.

By combining a series of transitions between long days and short days and combining these transitions with exposure to an appropriate temperature it is possible to predict and to control the time of breeding of selected groups of such worms. The procedures by which this may be achieved are large in number and can be set out as a series of protocols each of which may be designated to achieve a specified objective.

A number of such protocols are set out herein.

The examples of protocols given herein relate to the conditions and seasons of the Northern Hemisphere, in the Southern Hemisphere the effective dates, months and seasons would be the equivalent in the Southern Hemisphere solar cycle.

Described below are a series of protocols which may be used to effect some predetermined change in the time of sexual maturation and subsequently the time of breeding of marine worms of the family Nereidae and which may be worms of taxonomic designation Nereis (Neanthes) virens.

### Protocol I

Protocol I is a procedure to be initiated in a time of naturally occurring long days, which in the Northern Hemisphere may be June, to induce accelerated oocyte growth. It may be used to provide females of worms which may be Nereis (Neanthes) virens suitable for breeding or induction of spawning at times in advance of the natural breeding season and which may be expected to be possible in the period from December to March or earlier.

Animals having a birth date close to the natural breeding season (which in the event that the worms are Nereis (Neanthes) virens would be March/May) or which had a birth date outside that period and were maintained under ambient photoperiodic conditions as found in the natural environment from the time of birth or recovery from a system of preservation and which will have been maintained under such an ambient (natural north temperate) photoperiodic regime or a simulated photoperiodic regime with "long days" (photophase ) 12 hours) from the time of their birth to mid-September and "short days" (photophase ( 12 hours) from mid-September to mid-March and thereafter to the onset of the illustrated Protocol under "long days" at any suitable temperature for the encouragement of growth and preferably in the region of 20 °C.

The Protocol consists of a sequence of transfers from conditions of long days to conditions of short days from any period when the day length is long which may be for example May onwards at convenient (eg monthly) intervals. The temperature conditions to which the animals are transferred should be suitable for the development of the oocytes and, in the event that the females are female Nereis (Neanthes) virens the temperature may conveniently be 15 °C or lower and may by preference be in the region of 8 °C. The transfer from long days to short days may be by an instantaneous change from a photophase of 16 hours to a photophase of 8 hours or any sequence of progressive shortening of the effective photophase from greater than 12 to less than 12.

In the event that the animals to be treated by the Protocol are female Nereis (Neanthes) virens they may be selected by coelomic biopsy to have a modal oocyte diameter in the range of 40-120 µm at the time when the Protocol is initiated.

By the end of the period of naturally occurring long days all the animals subject to the Protocol will have been exposed to short day conditions and may be maintained at temperatures not greater than 15°C and preferably below 12°C until the time of breeding.

The animals will be re-examined by coelomic biopsy at any convenient interval, the purpose being to select those animals that have reached a stage of readiness for breeding.

Females with the modal oocyte diameter greater than 160 µm and a distinct clear cortical halo or having such other observable criteria to indicate a readiness to undergo fertilisation may be selected for breeding and/or the induction of spawning.

Male worms with developing germ cells at the tetrad stage of development or with freely dissociated spermatocyte or spermatid cells or with bundles of spermatozoa associated in sperm morulae or which have such other criteria as to suggest a readiness to take part in a fertilisation reaction and yield viable embryos may be selected for breeding and/or the induction of spawning.

### Protocol II

Protocol II is a Protocol to induce breeding in specimens of Nereis (Neanthes) virens and similar worms which have a semelparous life history and which do not in nature breed until two or more years old and which may not breed in captivity until they have reached such an age, and in which it would be desirable to induce breeding at some period in advance of that that would occur naturally as for example in May to July. The Protocol may be suitable to induce such breeding in female Nereis (Neanthes) virens that would not otherwise breed until they approach an age of two years.

The Protocol would be initiated soon after the naturally occurring date of the critical photoperiod which is 21 September in the Northern Hemisphere for Nereis (Neanthes) virens. The Protocol may be initiated in October or November and would be used to provide females suitable for breeding in the period from April to July of the following year. Females selected for treatment in a manner similar to that described for Protocol I herein will be selected by coelomic biopsy to have a modal oocyte diameter less than 120 µm or to be immature as being indicated by the absence of germ cells in the coelomic cavity in the early autumn period eg during October when it would normally be expected that such animals would not breed in the immediately following spring.

Suitable females for treatment with the Protocol may be female Nereis (Neanthes) virens and will generally have a birth date prior to May of the same year and, if reared in captivity, will have been maintained from birth under long day conditions at temperatures suitable for growth and preferably greater than 12°C and preferably at a temperature of 20°C.

According to the Protocol groups of such females will be transferred from long days to short days at some convenient intervals eg monthly intervals from September to January and simultaneously to temperatures of 12 °C or less than 12 °C or to conditions of decreasing temperature to reach a temperature less than 12 °C within eight weeks of transfer and at a stepwise rate of decrease in temperature of not more than 2 °C per week.

They will subsequently be maintained under conditions of short days and low temperature less than 12 °C.

From April onwards the animals will be examined by coelomic biopsy and females selected for breeding according to the size and the morphology of the coelomic oocytes and males selected by the appearance of tetrad stage germ cells or free spermatozoa in the coelomic fluid.

### Protocol III

Protocol III is designed to provide females for breeding in the period September to November.

Animals having a birth date in December (as may be achieved by other Protocols described herein) or having an effective birth date as determined by recovery of larvae from low temperature in or before December will have been reared under simulated ambient conditions for the first year of life as if the birth date was in April ie in long days for a period of about five to six months then to short days for a period of some five to six months and then to long days for a variable period according to the desired time of breeding and maintained under these conditions for a period of not less than one month but which may be a period of several months and which subsequently will be transferred to short days.

At the time of this transfer the animals will also be maintained at a temperature suitable for the maturation of germ cells such as a temperature of 12 °C or a temperature below 12 °C but above 4 °C.

Animals will be selected for the purposes of breeding by the criteria set out in Protocol I and Protocol II.

### Protocol IV

Protocol IV is designed to provide animals for breeding in the period from August to November.

The Protocol requires prior provision of juveniles which are themselves the offspring of one of the previously described Protocols or alternatively and by preference larvae whose effective birth date has been changed by a process of suspension of development such as may be achieved by a programme of low temperature dehydration.

The animals to be treated will have a birth date or date of recovery from a system of preservation prior to 31 January and the animals will be subjected to a long day and a temperature suitable for somatic growth which may preferably be 18 °C for a period of at least five months but which may be longer than five months after which period the animals would be transferred to conditions of short days and lower temperature which may conveniently be a temperature of 12 °C or lower than 12 °C and which may be a progressively reducing temperature. The animals will be maintained under the short day and low temperature conditions subsequently and examined by coelomic biopsy or similar treatment at convenient intervals.

Animals suitable for breeding will be expected to have developed within six months of the transfer to short day conditions and will be selected by the criteria described in Protocols I and II.

### Protocol V

Protocol V is an alternative means of achieving animals suitable for breeding in the period from July to September.

Animals whose birth dates are close to the natural breeding season in April will be exposed to a simulated regime of day length as if they were born in December ie they will be exposed to short days but the animals will be maintained in temperatures to encourage somatic growth and in the event that the animals are Nereis (Neanthes) virens by preference a temperature of 18 °C for a period of three months from birth. They will then be exposed to long days and temperatures to encourage somatic growth and in the event that the animals are Nereis (Neanthes) virens by preference a temperature of 18 °C for six months then returned to short days and a low temperature or preferably a temperature between 5 and 12 °C for a period of up to six months.

Following this sequence of transitions between photoperiods and regulation of temperature the animals will be selected for the purposes of breeding as described in the earlier described methods and Protocols.

The Protocols described illustrate some of the very many Protocols which may be designed to incorporate the specific knowledge and the innovative step in order to achieve the desired commercial objective.

In the event that the invention is used to provide juveniles of Nereis (Neanthes) virens throughout the year it will usually not be necessary for all of the Protocols described above to be used in order to achieve production of juvenile worms through the greater part of the year.

Animals selected as being suitable for breeding purposes by reference, in the case of females to the specific diameter of the coelomic oocytes or, in the case of males, to the presence of tetrad stage spermatocyte cells may be further treated to induce fertilisability or the onset of spawning.

Selected animals may also be decerebrated by the surgical removal of the prostomium or of the posterior parts of the prostomium or such other parts of the central nervous system as may be indicated by the existing scientific literature using such techniques as may reasonably be used to remove the centres of neurendocrine influence during the final stages of maturation.

The use of decerebration as a component in the novel and not previously envisaged circumstances described herein however has not been previously established as a technique for the realisation of the objective of breeding such worms outside the normal season of breeding activity.

Animals selected for breeding purposes may also be exposed to different conditions of maintenance in the periods after selection as may be expected to induce them to become sexually mature and that in the event that the selected females are female Nereis (Neanthes) virens such exposure may be to long day lengths and a temperature in the region of 20 °C for a period of one to a few weeks after the female has been selected and prior to the induction of artificial fertilisations.

Artificial fertilisations may be carried out by mixing oocytes that have developed a clear morphologically distinct halo with spermatozoa showing spontaneous activity when diluted in sea water. Spermatozoa that have been recovered from a cryopreservation system may be used.

The animals may be fed a pelletised diet with high lipid and high protein content at a rate of 2.5% body weight per day until some two months prior to the anticipated breeding date when, in the event that the animals are members of the family Nereidae the diet may be reduced progressively to zero.

Specific examples of the aspects of a number of the specified Protocols are given below.

### Example 1 - Induction of early sexual maturation (Protocol I)

### Step 1

Specimens of Nereis (Neanthes) virens were selected from stocks of females raised in captivity in a temperature regime of about 18 °C in beds of sand supplied with running sea water held constantly at this temperature by mixing natural sea water with that derived from a power station outfall that power station using sea water as a condenser coolant, and additionally maintained under natural ambient photoperiodic conditions from birth until the second June following their birth which was approximately 15 months earlier.

### Step 2

Samples of coelomic fluid were obtained by coelomic biopsy and in the case of females selected for the process and for the control treatment if the mean oocyte diameter was in the range 100-130 microns or, in the case of males, if spermatogonial platelets were present in the coelomic fluid.

### Step 3

The animals subjected to the process were placed into a short day photoperiodic treatment by enclosing them in concrete tanks with a sand substrate inside a building with wooden partitions and inside which there were tungsten lights subjected to time control and such that the light illuminated the beds of sand in which the worms were living and in the short day treatment the lights were switched on for a period of eight hours in each 24 hour period and at other times were switched off there being no other significant light entering the sheds and illuminating the beds of sand and the temperature of the sea water supplied to the tanks was no greater than 15 °C.

For the purposes of comparison another group of animals selected at the same time and of the same stock was maintained under essentially similar conditions but the period of illumination was that of the ambient photoperiodic conditions being determined by the time of sunrise and sunset at that latitude at that time of year.

### Step 4

Coelomic samples were taken in October some three months after the time of transfer to short day conditions. Approximately 50 oocytes were measured from each animal subjected to the treatment or treated as a control group.

A summary of the results is presented in Table 1 which shows that the mean oocyte diameter found in the animals subjected to the process was significantly greater than that of the animals not subjected to the process (see Table 1).

This was evidence that the growth of the oocytes in the treated females was significantly greater than that in the non-treated animals. The animals were subsequently maintained in a photoperiod with short days and at a temperature less than 12°C.

By 21 December all of the animals subjected to the experimental Protocol had proceeded through the stage of sexual maturation, had spawned and died as is normal following the sexual maturation and spawning of these animals. None of the animals maintained under the ambient conditions photoperiodic conditions had completed sexual maturation and spawned at this time.

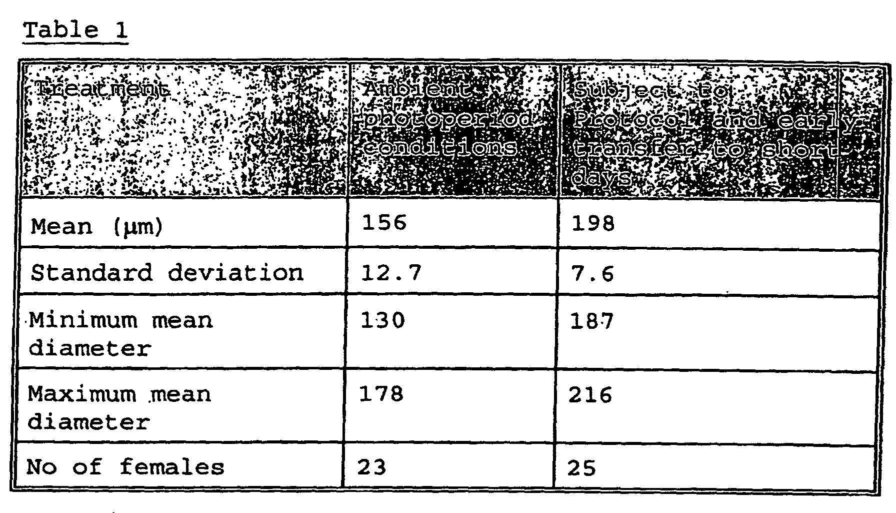

Table 1 - Mean oocyte diameter of the short day and ambient groups in October as a result of the treatment in Example 1.

### Example 2 - Photoperiod

The following example illustrates the response of females to a variety of photoperiodic manipulations as may be included in a number of the above Protocols.

### Step 1 - Maintenance and origin of the worms

The animals illustrating the process in this example were specimens of Nereis (Neanthes) virens raised in captivity in concrete beds supplied with sea water at an approximately constant temperature of 18 °C and fed on a proprietary diet and subjected to ambient photoperiods from birth to the onset of the Protocol treatments. The animals were all offspring of a single fecund mating known by the Inventors as genetic group 8.2 and had the same birth date of January 1994.

### Step 2 - Selection of the worms

The animals were selected in November 1994 and at that time they were all observed to lack either coelomic oocytes or spermatocyte platelets in the coelomic fluid which is an indication that they would not under normal circumstances or the circumstances of culture at 18 °C be expected to breed in the following Spring. Nereis (Neanthes) virens is a semelparous organism that breeds only once, and that breeding being normally in March or May would be expected to complete the processes of vitellogenesis during the period from September to December in the Autumn preceding the year in which the individuals would complete sexual maturation and thus be ready for the purposes of breeding or ready to complete the process of spawning in the following Spring.

### Step 3 - Maintenance in a specific photoperiodic regime

The animals selected for the procedure were introduced to plastic boxes supplied with sea water from a recirculating sea water system in a marine laboratory each box being inside a wooden construction fitted with fluorescent tube lights controlled by electronically operated time switches. The constructions being such that no other light illuminated the plastic boxes inside which the worms were held. The plastic boxes were supplied with a substrate of sand in which the animals made burrow systems and the animals were fed daily with a proprietary feed during times of natural daylight and when the fluorescent light tubes were switched on. The temperature of the sea water being supplied to the boxes was held throughout the period of operation of the photoperiodic Protocols at 17 °C.

Three different photoperiodic treatments are illustrated in this example: treatment A, treatment B and treatment C. The observations were made for the purpose of designing Protocols and the treatments do not necessarily relate to any one of the specific Protocols described herein.

### Treatment A

Selection of females in November 1994. Exposure to long day lengths in the equipment described at a temperature of 17 °C for a period of three months until February 1995 and subsequently maintained in short days but remaining at a temperature of 17 °C until examination on 24 May 1995.

### Treatment B

Selection of females in November 1994. Exposure to short day lengths from November to January, then subsequent exposure to long day lengths until examination on 24 May 1995 and held at a temperature of 17 °C throughout.

### Treatment C

Selection of females in November 1994. Exposure to short day lengths from November to April, then transfer to long days from April onwards and maintained at a temperature of 17 °C throughout.

Treatment C represents a simplified mimic of the ambient cycle of photoperiod but with constant temperature at 17 °C.

### Step 4

The effectiveness of the treatments was evaluated by means of measurements of oocyte frequency distributions for individually treated animals obtained by coelomic biopsy as in Example 1 and carried out in May 1995. Approximately 25 randomly selected oocytes in a sample of coelomic fluid of each female were measured using compound light microscopial observation.

The results are shown in Tables 2A, 2B and 2C which summarise the mean oocyte diameter data for animals subjected to each treatment.

**Table 2A**

| Female no | Sample | Mean oocyte diameter (microns) | Standard deviation |
|---|---|---|---|
| 1 | 29 | 169 | 5.18 |
| 2 | 28 | 171 | 5.82 |
| 3 | 26 | 173 | 4.79 |
| 4 | 26 | 176 | 6.58 |
| Mean | | 172 | |

**Table 2B**

| Female no | Sample | Mean oocyte diameter (microns) | Standard deviation |
|---|---|---|---|
| 1 | 29 | 137 | 9.68 |
| 2 | 30 | 130 | 10.16 |
| 3 | 25 | 143 | 6.85 |
| 4 | 28 | 140 | 6.49 |
| 5 | 27 | 136 | 8.21 |
| 6 | 29 | 126 | 9.06 |
| Mean | | 136 | |

**Table 2C**

| Female no | Sample | Mean oocyte diameter (microns) | Standard deviation |
|---|---|---|---|
| 1 | 31 | 149 | 13.06 |
| 2 | 25 | 157 | 8.53 |
| 3 | 28 | 141 | 30.94 |
| 4 | 30 | 168 | 3.61 |
| 5 | 29 | 166 | 5.48 |
| 6 | 25 | 152 | 21.64 |
| Mean | | 136 | |

The data show the clear divergence in the rate of oocyte development induced by the exposure to the different day length treatments at a constant temperature, the females held in Treatment A being substantially more mature than those held in Treatment B and Treatment C.

### Example 3 - Temperature regulation

The response of individual females to a change in day length is also influenced by the temperature at which the animals are maintained and therefore the Protocols devised and explained herein in some circumstances and for some purposes designate the temperature at which organisms should be maintained in certain specific circumstances. This example illustrates this aspect of a typical Protocol.

### Step 1

The animals illustrating the process in this example were specimens of Nereis (Neanthes) virens raised is captivity in concrete beds supplied with sea water at an approximately constant temperature of 18 °C and fed on a proprietary diet and subjected to ambient photoperiods from birth until the onset of Protocol treatments. The animals were all offspring of a single fecund mating known by the Inventors as genetic group 9.7 and had the same birth date, that birth date being 10 February 1995.

### Step 2

The animals were selected on 24 January 1996 using as the criterion for selection the observation of coelomic oocytes in a coelomic biopsy such that each animal selected was female and such that the modal and mean oocyte diameter of the coelomic oocytes were less than 100 microns.

### Step 3

The animals selected for the procedure were introduced to plastic boxes supplied with sea water from a recirculating sea water system in a marine laboratory, each box being inside a wooden construction fitted with fluorescent tube lights controlled by electronically operated time switches. The construction being such that no other light illuminated the plastic boxes inside which the worms were held. The plastic boxes were supplied with a substrate of sand in which the animals made burrow systems and the animals were fed daily with a proprietary feed during times of natural daylight and when the fluorescent light tubes were switched on. The temperature of the sea water being supplied to the boxes was initially 16 °C.

The animals were exposed to a period of six weeks in which the photoperiod was a simulation of long days with the photophase being 16 hours and the scutaphase being 8 hours in duration. After a period of six weeks the boxed animals were subdivided into two groups for the purposes of illustration of the effectiveness of treatments that are incorporated into specific Protocols are described herein. Both groups were from this time exposed to the same photoperiodic regime which was exposure to simulated short days in which the photophase was eight hours and the scutaphase 16 hours but one group (Group 3A) was supplied with sea water at 16 °C and the other group (Group 3B) was supplied with sea water that was progressively cooled at a rate of 3 °C each month ie 13 °C in March, 10 °C in April and 7 °C in May.

### Step 4

The animals were re-examined at intervals including examination on 20 May 1996 and the diameter of oocytes in samples of coelomic fluid obtained by biopsy was measured and recorded.

The results are summarised in Table 3.

| Sample Date | | | | | | | | |
|---|---|---|---|---|---|---|---|---|
| | 24.1.96 | | 1.3.96 | | 22.4.96 | | 20.5.96 | |
| Group | n | mean | n | mean | n | mean | n | mean |
| 3A (warm) | 10 | 76 | 9 | 107 | 6 | 104 | 6 | 107 |
| 3B (cool) | 8 | 77 | 8 | 107 | 7 | 126 | 7 | 140 |

The animals have continued their sexual development and those in Group 3B are substantially advanced in sexual development and are indicating a readiness for spawning in July 1996.

### Example 4 - Decerebration

In the event that the animals selected for the operation of the invented process are specimens of Nereis (Neanthes) virens.

### Step 1

The animals illustrated in the process in this example were specimens of Nereis (Neanthes) virens raised in captivity in concrete beds supplied with sea water at an approximately constant temperature of 18 °C and fed on a proprietary diet and subjected to ambient photoperiods from birth until the onset of the Protocol treatments. The animals were all offspring of a single fecund mating known by the Inventors as genetic group 9.7 and had the same birth date, that birth date being 10 February 1995.

### Step 2

The animals were selected on 8 May 1996 such that each animal selected was female and such that the modal and mean oocyte diameter of the coelomic oocytes was between 100 and 120 microns. Animals with such oocytes subject to the photoperiodic conditions that occur in nature would cease to develop the oocytes and would not be expected to breed until the Spring of the following year, such timing being determined by the sequence of photoperiod and temperature as has been observed by the Inventors.

### Step 3

The animals selected for the procedure were introduced to plastic boxes supplied with sea water from a recirculating sea water system in a marine laboratory, each box being inside a wooden construction fitted with fluorescent tube lights controlled by electronically operated time switches. The constructions being such that no other light illuminated the plastic boxes inside which the worms were held. The plastic boxes were supplied with a substrate of sand on which the animals made burrow systems and the animals were fed daily with a proprietary feed during times of natural daylight and when the fluorescent light tubes were switched on. The temperature of the sea water being supplied to the boxes was in some cases 16 °C or alternatively 10 °C or 7 °C as set out in the summary of the treatments set out below.

Additionally, one group of animals was subjected to a decerebration procedure in which the posterior part of the prostomium containing the supra-oesophageal ganglion was removed by surgical removal of the tissues in the prostomium in the region defined by the posterior margin of the prostomium and a line passing in front of the anterior pair of prostomial eyes. The surgical procedure was carried out in sterile sea water using iridectomy scissors.

The animals were allocated to five experimental groupings as follows:
- Group A -: exposure to short days and to a temperature of 10 °C
- Group B -: exposure to short days and to a temperature of 16 °C
- Group C -: exposure to long days and to a temperature of 10 °C
- Group D -: exposure to long days and to a temperature of 16 °C
- Group E -: exposure to short days and to a temperature of 7 °C.

The development of the oocytes was followed in all the groups and differences observed between them as summarised in Table 4.

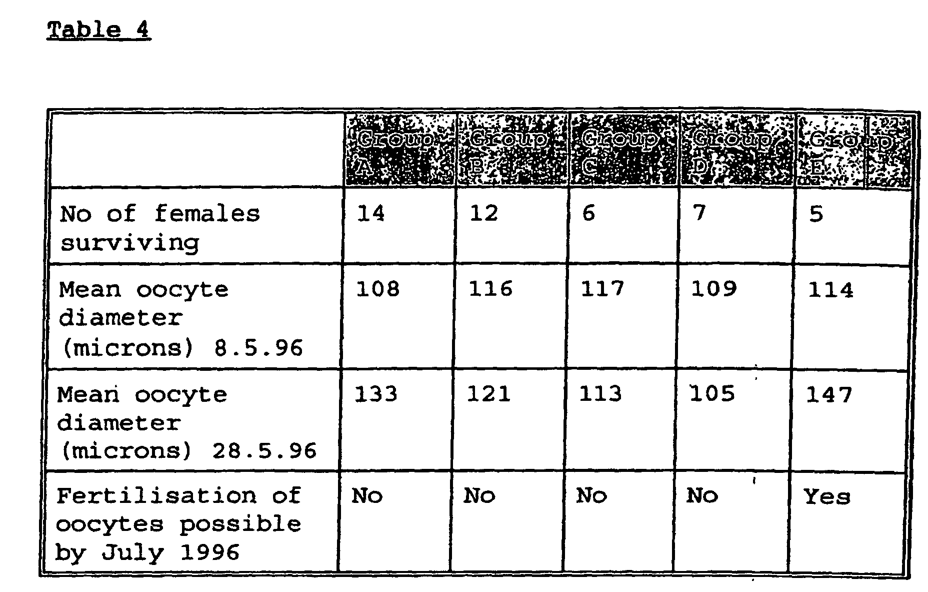

The observations illustrate the effectiveness of combining decerebration procedures with certain photoperiod and temperature sequences in order to achieve fertilisations of oocytes outside the breeding season.

In this instance the fertilisations were carried out using spermatozoa of Nereis (Neanthes) virens recovered from liquid Nitrogen that had been preserved by a cryopreservation process.

## Claims

1. A method of inducing the time of sexual maturation of polychaete worms of the *Nereidae* family outside the natural time for maturation, comprising exposing the worms to a photoperiodic regime different to the natural photoperiodic regime, wherein the photoperiodic regime to which the worms is subjected mimics a natural photoperiodic regime but takes place at a different time of year to the natural photoperiodic regime being copied, and wherein the worms are maintained in a controlled temperature regime.

2. A method in accordance with Claim 1 in which the worms have a date of birth outside the natural breeding season.

3. A method in accordance with Claim 1 in which the worms are recovered from a preservation system.

4. A method in accordance with Claim 1 in which the photoperiodic regime to which the worm is exposed is displaced by three months relative to the natural photoperiodic regime.

5. A method in accordance with Claim 1 in which the photoperiodic regime to which the worm is exposed is displaced by six months relative to the natural photoperiodic regime.

6. A method in accordance with any one of Claims 1 to 3 in which the photoperiodic regime to which the worms are exposed is one in which the duration of a period of short days is greater than that occurring in the natural photoperiodic regime.

7. A method in accordance with any one of Claims 1 to 3 in which the photoperiodic regime to which the worms are exposed is one in which the duration of a period of long days is greater than that occurring in the natural photoperiodic regime.

8. A method in accordance with any one of Claims 1 to 7 in which during a period of short days the photophase is constant.

9. A method in accordance with Claim 1 wherein polychaete worms of the Nereidae family having a natural breeding season in the Spring are induced to become sexually mature at a time in advance of that by exposing the worms to a change from long days to short days in advance of the time when the change from long days to short days occurs in the natural photoperiodic regime.

10. A method in accordance with any preceding Claim in which the worms are of the species Nereis (Neanthes) virens.

11. A method of accordance with any one of Claims 1 to 10, in which the worms are decerebrated as part of the method.

12. A method in accordance with any one of Claims 1 to 11, in which the worms are subjected to temperatures of 5-10°C.

## Patentansprüche

1. Ein Verfahren zum Induzieren der Zeit sexueller Reifung von Polychaeten der Familie *Nereidae* außerhalb der natürlichen Zeit der Reifung, welches beinhaltet, dass die Würmer einem photoperiodischen Regime, das sich vom natürlichen photoperiodischen Regime unterscheidet, ausgesetzt werden, wobei das photoperiodische Regime, dem die Würmer unterzogen werden, ein natürliches photoperiodisches Regime imitiert, aber zu einer Zeit im Jahr, die sich von dem kopierten natürlichen photoperiodischen Regime unterscheidet, stattfindet, und wobei die Würmer in einem kontrollierten Temperaturregime gehalten werden.

2. Verfahren gemäß Anspruch 1, bei dem die Würmer ein Geburtsdatum aufweisen, das außerhalb der natürlichen Fortpflanzungssaison liegt.

3. Verfahren gemäß Anspruch 1, bei dem die Würmer aus einem Konservierungssystem zurückgebracht werden.

4. Verfahren gemäß Anspruch 1, bei dem das photoperiodische Regime, dem der Wurm ausgesetzt wird, relativ zum natürlichen photoperiodischen Regime um drei Monate verschoben ist.

5. Verfahren gemäß Anspruch 1, bei dem das photoperiodische Regime, dem der Wurm ausgesetzt wird, relativ zum natürlichen photoperiodischen Regime um sechs Monate verschoben ist.

6. Verfahren gemäß einem der Ansprüche 1 bis 3, bei dem das photoperiodische Regime, dem die Würmer ausgesetzt werden, eines ist, bei dem die Dauer einer Periode kurzer Tage größer ist als die, die im natürlichen photoperiodischen Regime vorkommt.

7. Verfahren gemäß einem der Ansprüche 1 bis 3, bei dem das photoperiodische Regime, dem die Würmer ausgesetzt werden, eines ist, bei dem die Dauer einer Periode langer Tage größer ist als die, die im natürlichen photoperiodischen Regime vorkommt.

8. Verfahren gemäß einem der Ansprüche 1 bis 7, bei dem die Lichtphase während einer Periode kurzer Tage konstant ist.

9. Verfahren gemäß Anspruch 1, wobei Polychaeten der Familie *Nereidae*, die eine natürliche Fortpflanzungssaison im Frühling aufweisen, induziert werden, zu einer Zeit davor sexuelle Reife zu erlangen, indem die Würmer einem Wechsel von langen Tagen zu kurzen Tagen vor der Zeit, wenn der Wechsel von langen Tagen zu kurzen Tagen im natürlichen photoperiodischen Regime vorkommt, unterzogen werden.

10. Verfahren gemäß einem der vorhergehenden Ansprüche, bei dem die Würmer der Spezies Nereis (Neanthes) virens angehören.

11. Verfahren gemäß einem der Ansprüche 1 bis 10, bei dem die Würmer als Teil des Verfahrens dezerebriert werden.

12. Verfahren gemäß einem der Ansprüche 1 bis 11, bei dem die Würmer Temperaturen von 5 - 10 °C unterzogen werden.

## Revendications

1. Un procédé pour amener le moment de maturation sexuelle de vers polychètes de la famille *Nereidae* à ne pas être compris dans le moment nature) pour la maturation, comportant l'exposition des vers à un régime photopériodique différent du régime photopériodique naturel, dans lequel le régime photopériodique auquel les vers sont soumis imite un régime photopériodique naturel mais a lieu à un moment de l'année différent du régime photopériodique naturel étant copié, et dans lequel les vers sont maintenus dans un régime de température régulée.

2. Un procédé conformément à la revendication 1 dans lequel les vers ont une date de naissance non comprise dans la saison de reproduction naturelle.

3. Un procédé conformément à la revendication 1 dans lequel les vers sont récupérés d'un système de conservation.

4. Un procédé conformément à la revendication 1 dans lequel le régime photopériodique auquel le ver est exposé est décalé de trois mois par rapport au régime photopériodique naturel.

5. Un procédé conformément à la revendication 1 dans lequel le régime photopériodique auquel le ver est exposé est décalé de six mois par rapport au régime photopériodique naturel.

6. Un procédé conformément à n'importe laquelle des revendications 1 à 3 dans lequel le régime photopériodique auquel les vers sont exposés est un régime dans lequel la durée d'une période de jours courts est supérieure à celle se produisant dans le régime << photopériodique naturel.

7. Un procédé conformément à n'importe laquelle des revendications 1 à 3 dans lequel le régime photopériodique auquel les vers sont exposés est un régime dans lequel la durée d'une période de jours longs est supérieure à celle se produisant dans le régime photopériodique naturel.

8. Un procédé conformément à n'importe laquelle des revendications 1 à 7 dans lequel la photophase est constante durant une période de jours courts.

9. Un procédé conformément à la revendication 1 dans lequel des vers polychètes de la famille *Nereidae* ayant une saison de reproduction naturelle au printemps sont amenés à venir à maturité sexuelle à un moment antérieur à celle-ci en exposant les vers à un changement de jours longs à jours courts antérieur au moment où le changement de jours longs à jours courts se produit dans le régime photopériodique naturel.

10. Un procédé conformément à n'importe quelle revendication précédente dans lequel les vers sont de l'espèce Nereis (Neanthes) virens.

11. Un procédé conformément à n'importe laquelle des revendications 1 à 10, dans lequel les vers sont décérébrés selon une partie du procédé.

12. Un procédé conformément à n'importe laquelle des revendications 1 à 11, dans lequel les vers sont soumis à des températures de 5 à 10 °C.
